Europäisches Patentamt

European Patent Office

Office européen des brevets

(19)

(11) Veröffentlichungsnummer : **0 355 470 B1**

(12)     **EUROPÄISCHE PATENTSCHRIFT**

(45) Veröffentlichungstag der Patentschrift :
**14.10.92 Patentblatt 92/42**

(51) Int. Cl.$^5$ : **A61K 9/22, A61K 9/26**

(21) Anmeldenummer : **89113925.5**

(22) Anmeldetag : **28.07.89**

(54) **Retardierte Arzneiform und Verfahren zu ihrer Herstellung.**

Die Akte enthält technische Angaben, die nach
dem Eingang der Anmeldung eingereicht
wurden und die nicht in dieser Patentschrift
enthalten sind.

(30) Priorität : **11.08.88 DE 3827214**

(43) Veröffentlichungstag der Anmeldung :
**28.02.90 Patentblatt 90/09**

(45) Bekanntmachung des Hinweises auf die
Patenterteilung :
**14.10.92 Patentblatt 92/42**

(84) Benannte Vertragsstaaten :
**AT BE CH DE ES FR GB IT LI NL SE**

(56) Entgegenhaltungen :
**GB-A- 2 187 643**

(56) Entgegenhaltungen :
**US-A- 2 987 445
US-A- 3 087 860
JOURNAL OF PHARMACEUTICAL SCIENCES,
Band 60, Nr. 2, Februar 1971, Seiten 212-215,
American Pharmaceutical Association,Washington, US; B. FARHADIEH et al.: "Drug release from methyl acrylate-methyl methacrylate
copolymer matrix II: Control of releaserate by
exposure to acetone vapor"**

(73) Patentinhaber : **Röhm GmbH
Kirschenallee Postfach 4242
W-6100 Darmstadt 1 (DE)**

(72) Erfinder : **Nürnberg, Eberhard, Prof. Dr.
Ruhsteinweg 18
W-8525 Uttenreuth-Weiher (DE)**
Erfinder : **Pfeuffer, Josef
Festungsstrasse 25
W-8742 Band Könighofen (DE)**

EP 0 355 470 B1

## Beschreibung

Die Erfindung betrifft neue Arzneiformen mit verzögerter Wirkstoffreisetzung aus einer Matrix und ein Verfahren zu ihrer Herstellung. Die Behandlung einer Arzneiform in der Weise, daß sie den Wirkstoff an eine umgebende physiologische Flüssigkeit langsamer als ohne diese Vorbehandlung abgibt, wird als "Retardierung" bezeichnet. Im Gegensatz zu überzogenen Arzneiformen, bei denen der Überzug die verzögerte Wirkstofffreisetzung bewirkt, beruht die verzögerte Freisetzung hier auf der Matrix selbst und kommt infolgedessen auch an beliebigen Bruchstücken der Matrix zur Wirkung.

## Stand der Technik

Werden Arzneiformen aus einer Mischung eines pulverförmigen Wirkstoffes und eines pulverförmigen Hilfsstoffs gepreßt, so setzen sie den Wirkstoff in einem wäßrigen Medium schnell frei. Nach US 3 087 860 wird die Freisetzungsgeschwindigkeit verzögert, wenn man eine solche Arzneiform den Dämpfen einer organischen Flüssigkeit aussetzt, die ein Lösemittel für den Hilfsstoff ist. Das Lösemittel wird von dem Hilfsstoff aus der Dampfphase aufgenommen und bewirkt das Zusammenfließen der Hilfsstoffteilchen zu einer zusammenhängenden Matrix, die die Wirkstoffteilchen vollkommen einschließt. B. Farhadieh, S. Borodkin und J.D. Buddenhagen (Journal of Pharmaceutical Sciences, Vol.60, 1971, S.209-212) haben derartige Matrixtabletten, die ein Methylacrylat-Methylmethacrylat-Mischpolymerisat als Hilfsstoff enthielten und mit Acetondampf behandelt worden waren, untersucht und eine deutliche Verminderung der Freisetzungsgeschwindigkeit festgestellt.

Das Retardierungsverfahren unter Anwendung von Aceton hat jedoch einige schwerwiegende Nachteile. So hat sich der Verzögerungseffekt als schlecht reproduzierbar erwiesen und ändert sich beim Lagern. Er ist überdies von der Größe der behandelten Arzneiformen abhängig und nimmt innerhalb der Arzneiform von außen nach innen ab. Das hat zur Folge, daß Bruchstücke der behandelten Arzneiformen eine höhere Freisetzungsgeschwindigkeit zeigen als ungeteilte Arzneiformen. Darüberhinaus hat es sich als schwierig erwiesen, das Aceton aus den behandelten Arzneiformen wieder so weitgehend zu entfernen, wie es nach den pharmakologischen Reinheitsanforderungen notwendig ist. Dieser Nachteil wiegt vor allem bei Aceton, Chlorkohlenwasserstoffen oder Aromaten schwer, weil sie pharmakologisch nicht indifferent und mehr oder weniger toxisch sind.

## Aufgabe und Lösung

Der Erfindung liegt die Aufgabe zugrunde, das Verfahren de Freigabeverzögerung von Arzneiformen, die eine gepreßte Matrix aus pulverförmigen Hilfsstoffen und Wirkstoffen enthalten, durch Einwirkung von Dämpfen organischer Flüssigkeiten dahingehend zu verbessern, daß die erwähnten Nachteile vermieden, eine durch die Einwirkungsdauer steuerbare, reproduzierbare Retardierung in relativ kurzen Behandlungszeiten erreicht und die organische Flüssigkeit aus den behandelten Arzneiformen schnell desorbiert wird und organische Flüssigkeiten von geringer Toxizität eingesetzt werden können.

Es zeigte sich, daß diese Aufgabe unter Verwendung guter Lösemittel für den Hilfsstoff, wie Chloroform, Dichlormethan, Aceton oder Toluol, nicht befriedigend gelöst werden konnte. Überraschenderweise ergaben aber niedere Alkohole die erwünschte Wirkung, obwohl die Hilfsstoffe aus der Gruppe der nicht im Magensaft löslichen Acrylpolymere und Vinyesterpolymere darin oft nur quellbar, aber nicht oder nur schwer löslich sind.

## Wirkungsweise und Vorteile der Erfindung

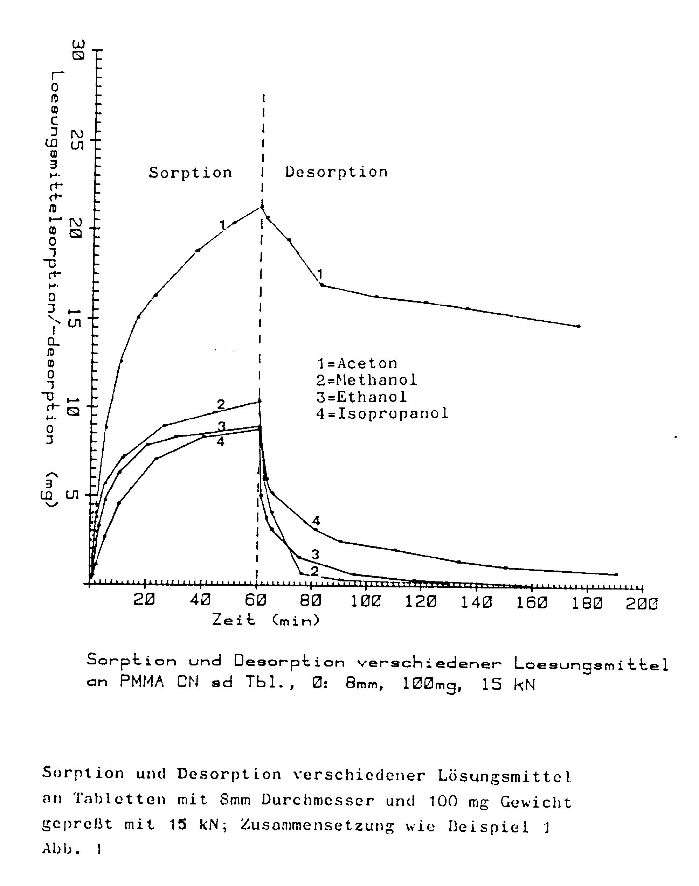

Der durch die Erfindung erzielbare Vorteil läßt sich am Beispiel der Einwirkung verschiedener niederer Alkanole im Vergleich zu Aceton aufzeigen. Figur 1 zeigt die Lösemittelaufnahme aus der Dampfphase in einer gepreßten Tablette von 8 mm Durchmesser und 100 mg Gewicht, die 10 % Kochsalz als Testwirkstoff in einer Matrix aus einem Mischpolymerisat aus gleichen Teilen Methylmethacrylat und Äthylacrylat enthält. Die Tabletten wurden 60 min lang dem Lösemitteldampf ausgesetzt und anschließend getrocknet. Aceton wurde etwa doppelt so schnell aufgenommen wie Methanol, Äthanol und Isopropylalkohol, aber beim Trocknen nur sehr langsam desorbiert, während die niederen Alkohole innerhalb von 1 bis 2 Stunden fast vollständig entfernt waren. Der verbleibende Rest ist - außer bei Methanoltoxikologisch unbedenklich.

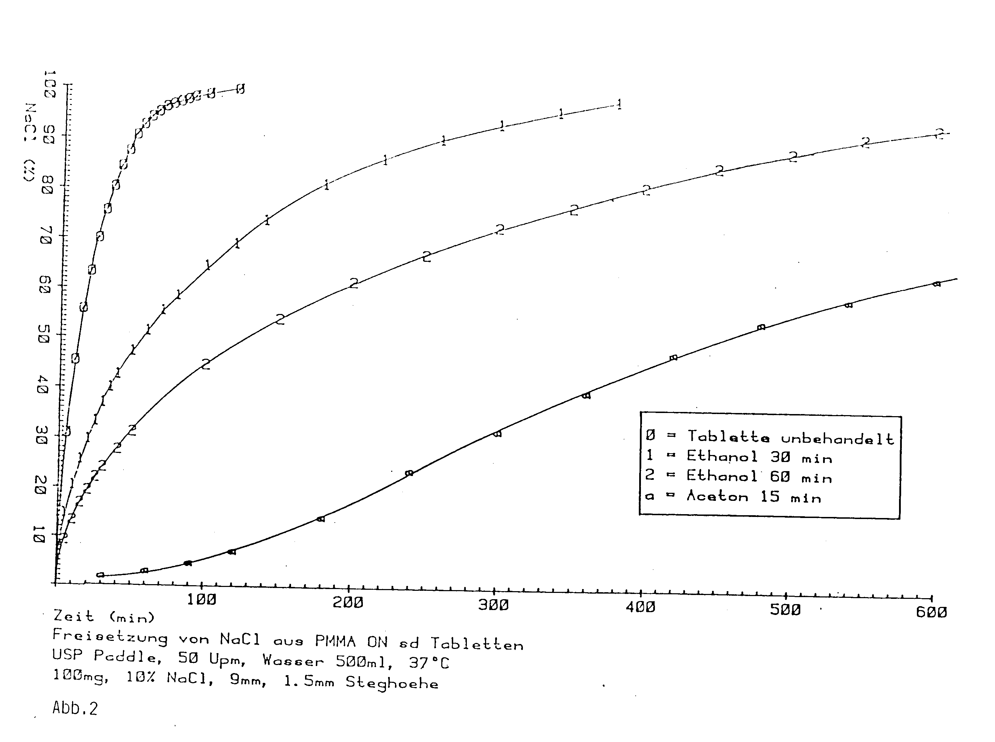

Figur 2 zeigt die Freisetzung von Kochsalz aus Tabletten der beschriebenen Art, die unbehandelt (0), 30 min (1) oder 60 min (2) mit Äthanoldampf oder 15 min mit Acetondampf (a) behandelt worden waren. Die unbehandelte Tablette gibt den Testwirkstoff unerwünscht schnell ab. Durch die Behandlung mit Äthanoldampf während 30 bzw. 60 min wurde eine deutlich gestaffelte Retardierung erreicht, mit der sich eine vollständige Wirkstofffreisetzung während der normalen Aufenthaltsdauer im Magen-Darm-Trakt einstellen läßt. Dagegen führt die Behandlung mit Acetondampf schon in 15 min zu einer so starken Retardierung, daß eine Freisetzung von mehr als 50 % des Wirkstoffes beim Durchgang durch Magen und Darm kaum zu erreichen wäre.

Es hat den Anschein, als ob die nur begrenzte Lösefähigkeit der niederen Alkohole gegenüber den verwendeten Hilfsstoffen eine wichtige Voraussetzung für die beobachtete Wirkung ist. Aceton und Chlorkohlenwasserstoffe, die gute Lösemittel für die Hilfsstoffe sind und das diesem Grund in der US 3 087 860 empfohlen werden, führen sehr schnell zu einer Anquellung, Lösung und Verklebung der Hilfsstoffteilchen an der Oberfläche der Arzneiformen. Sie fließen zu einer geschlossenen Schlicht zusammen, die das weitere Eindringen der Lösemitteldämpfe in das Innere der Arzneiform behindert. Auf diese Weise entsteht ein Konzentrationsgefälle von außen nach innen, das über die Dauer der Behandlung hinaus bestehen bleibt. Nach Abschluß der Behandlung kann das Lösemittel nur beschränkt desorbieren. Im Laufe der Zeit kommt es zu einem Ausgleich der Lösemittelkonzentration innerhalb der Arneiform, was vor allem die unerwünschte Wirkung hat, daß sich das Freisetzungsverhalten beim Lagern ändert.

Demgegenüber werden die Hilfsstoffteilchen durch die eindringenden Dämpfe der niederen Alkohole nur angequollen und erweicht, so daß sie an den gegenseitigen Berührungspunkten miteinander verkleben. Die beim Verpressen de pulverförmigen Ausgangsstoffe gebildete Matrix bleibt während der Dampfbehandlung porös, auch wenn sich die Porengröße verringert. Der Alkoholdampf kann infolgedessen in dem Maße von außen nachströmen, wie er im Innern absorbiert wird. Wegen der offenporigen Struktur erfolgt die Absorption schnell und weitgehend gleichmäßig über die ganze Matrix einer Arzneiform. Darauf beruhen die Vorteile de Erfindung: Es gibt kein Konzentrationsgefälle, das zu eine Eigenschaftsänderung beim Lagern führen könnte. Die Verdichtung der Matrix und die dadurch bewirkte Verzögerung der Wirkstofffreigabe treten über die ganze Matrix gleichmäßig ein, so daß nach der Behandlung absichtlich oder unabsichtlich entstandene Bruchstücke - abgesehen von einem kleinen, auf den unterschiedlich langen Diffusionswegen beruhenden Effektdie gleiche Freisetzungscharakteristik zeigen wie unversehrte Arzneiformen. Die Desorption des Alkohols nach der Behandlung gelingt ebenso schnell und gleichmäßig wie zuvor die Absorption. Daher ist die Gefahr unzulässig hoher Lösemittelrückstände in den erfindungsgemäßen Arzneiformen gering.

Wenn trotzdem Reste des Alkohols, insbesondere des bevorzugt eingesetzten Äthylakohols, in der Arzneiform verbleiben, so ist dies vom pharmakologischen Standpunkt wegen der geringen Toxizität der Alkohole in der Regel unbedenklich. Lediglich Methanol bildet in dieser Hinsicht eine Ausnahme.

Die Gasphase

Als niedere Alkohole kommen vor allen die primären, sekundären oder tertiären Alkanole mit 1 bis 4, vorzugsweise 2 bis 4 Kohlenstoffatomen in Betracht. Bevorzugt sind Propanol-1, Propanol-2, n-Butanol, Butanol-2, tert. Butanol und besonders Äthanol.

Es ist vorteilhaft, wenn die Gasphase, mit der die Arzneiformen behandelt werden, im wesentlichen allein aus dem Dampf des Alkohols besteht. In diesem Falle strömt die Gasphase in dem Maße in die Poren der Arzneiform nach, wie der Alkohol im Innern absorbiert wird. Enthält die Gasphase dagegen wesentliche Anteile an nicht absorbierbaren Inertgasen, so sammeln sich diese in den Poren an und weiterer Alkoholdampf kann nur durch Diffusion in die Poren nachströmen. Zudem ergeben die relativ langsamen Diffusionsprozesse bis zur Einstellung eines homogenen Gleichgewichts der Mischgasphase Probleme der Reproduzierbarkeit. Es ist deshalb vorteilhaft, den Anteil des Alkoholdampfes an der Gasphase so hoch wie möglich zu halten. Auf jeden Fall soll der Alkohol-Partialdruck überwiegen. In der Praxis liegt der Alkoholanteil, bezogen auf den Partialdruck, vorwiegend im Bereich von 80 bis 100 %. Außer Luft, die sich nur mit großem technischem Aufwand vollständig aus der Gasphase ausschließen läßt, tritt als Nebenbestandteil der Gasphase vor allem Wasserdampf auf.

Der absolute Druck der Gasphase ist dagegen von geringerer Bedeutung. Er kann z.B. im Bereich von 20 bis 1000 mbar oder gegebenenfalls darüber liegen. Bei niedrigen Alkohol-Partialdrücken kann durch längere Dampfeinwirkungsdauer der gleiche Effekt wie bei hohem Partialdruck erreicht werden. Um einen vorteilhaften Alkohol-Partialdruck zu erreichen, kann bei erhöhter Temperatur gearbeitet werden. Der bevorzugte Temperaturbereich liegt zwischen 18 und 50°C. Die Erweichungstemperatur des Hilfsstoffes muß berücksichtigt werden; eine zu starke Erweichung bei der Aufnahme des Lösemitteldampfes wäre nachteilig und kann durch eine niedrigere Behandlungstemperatur vermieden werden.

Der Hilfsstoff

hat die Aufgabe, beim Pressen zusammen mit dem Wirkstoff ein festes Komprimat zu bilden, worin die Hilfsstoffteilchen einander so berühren, daß sie bei der erfindungsgemäßen Behandlung mit Alkoholdampf eine zusammenhängende, aber poröse Matrix bilden. Er wird so gewählt, daß er in den Körperflüssigkeiten, mit denen die Matrix in der Zeit bis Freisetzung der Hauptmenge des Wirkstoffes in Berührung tritt, nicht gelöst wird. Bei oral applizierten Arzneiformen ist das der Magen- und gegebenenfalls Darmsaft. In der Regel ist es nicht nachteilig oder sogar vorteilhaft, wenn sich die Matrix nach Freisetzung des überwiegenden Teils des Wirkstoffes löst. Der Hilfsstoff braucht also in den unteren Darmabschnitten nicht in jedem Fall unlöslich zu sein. Für Arzneiformen, die in anderen Körperöffnungen, wie der Mundhöhle oder im Augenlid, oder als Implantat angewendet werden, genügt die Unlöslichkeit in den dort auftretenden Körperflüssigkeiten.

Als Hilfsstoffe werden physiologisch unbedenkliche Acrylester- und Vinylester-Polymerisate verwendet, die wenigstens im Magensaft, aber bevorzugt auch im Darmsaft unlöslich sind. Eine für die Erfindung wesentliche Eigenschaft dieser Polymerisate ist ihre Fähigkeit, niedere Alkohole unter Quellung und Erweichung reversibel aufzunehmen.

Solange die Matrix des erfindungsgemäß behandelten Komprimats ungelöst ist, wird der Wirkstoff allein durch Diffusion freigesetzt. Im Falle eines magen- und darmsaftunlöslichen Hilfsstoffes wird die Matrix nach Abgabe des Wirkstoffes äußerlich unverändert ausgeschieden. Eine Lösung oder Quellung der Matrix im Magen- oder Darmsaft würde die Freigabecharakteristik beeinflussen und kann insoweit zugelassen werden, wie solche Änderungen erwünscht sind. Wenn die Matrix erst bei steigendem pH-Wert in den unteren Darmabschnitten löslich wird, ist der Wirkstoff in der Regel schon vorher zum größten Teil durch Diffusion freigesetzt worden. Die Freisetzung der Restmenge des Wirkstoffes geschieht dann zunehmend durch die allmähliche Auflösung der Matrix.

Die bevorzugten, magen- und darmsaftunlöslichen Hilfsstoffe sind überwiegend oder ganz aus Acrylestern oder Vinylestern aufgebaut. Als Acrylester sind vor allem die Alkylacrylate und Alkylmethacrylate mit 1 bis 8, vorzugsweise 1 bis 4 C-Atomen im Alkylrest geeignet. Typische Vinylester sind Vinylacetat, Vinylpropionat und Vinylbutyrat. Sofern wasserunlösliche Comonomere, wie Styrol, Äthylen, Propylen oder Vinylchlorid, verwendet werden, ist das Copolymerisat in Magen- und Darmsaft unlöslich. Bevorzugte Acrylpolymere sind Homo- oder Copolymerisate aus wenigstens 30 Gew.-%, vorzugsweise 50 bis 100 Gew.-%, eines Alkylesters der Acryl- oder Methacrylsäure mit 1 bis 8 C-Atomen im Alkylrest und bis zu 70 Gew.-% eines damit mischpolymerisierbaren, alpha-beta-äthylenisch ungesättigten comonomeren.

Man erhält darmsaftlösliche Copolymerisate, wenn das Copolymerisat unvernetzt ist und zu 30 bis 70 Gew.% aus comonomeren mit Carboxyl- bzw. Carboxylatgruppen aufgebaut ist. Dazu gehören z.B. Acryl- und Methacrylsäure, Malein-, Fumar- und Itakonsäure. Hydroxyalkylester der Acryl- oder Methacrylsäure fördern die Löslichkeit. Bevorzugte Acrylpolymere dieser Art enthalten als Comonomerkomponente Einheiten von Acryl- oder Methacrylsäure und/oder von deren Hydroxyalkylestern, wobei deren Alkylreste 2 bis 6 C-Atome enthalten.

Der Hilfsstoff wird in Form eines preßbaren Pulvers eingesetzt. Die Pulverkörnchen sind vorzugsweise 50 bis 250 Mikrometer groß. sie können aus einem Substanzpolymerisat durch Mahlen oder aus einer wäßrigen Dispersion eines Emulsionspolymerisats durch Sprühtrocknen erhalten worden sein. Perlpolymerisate können gegebenenfalls in unveränderter Form eingesetzt werden.

**Der Wirkstoff**

wird ebenfalls in Pulverform eingesetzt, kann aber in gröberer Teilchengröße vorliegen, beispielsweise 0,1 bis 0,8 mm. Die Erfindung eignet sich für jeden kristallisierten oder amorphen Wirkstoff, dessen verzögerte Freisetzung im Magen-Darmtrakt erwünscht ist, ebenso für Wirkstoffmischungen. Typische Beispiele solcher Wirkstoffe sind Acetylsalicylsäure, nicht steroidale Antirheumatika, Antihypertensiva, Herzglykoside, Antiasthmatika, Sedativa, Stimulantien, Antibiotika, Spasmolytika, Hormone und Psychopharmaka. Der Anteil des Wirkstoffes kann z.B. 1 bis 90 Gew.-% des zu verpressenden Pulvergemisches ausmachen.

Darin können auch weitere übliche Zusatzstoffe, wie hochdisperse Kieselsäure, Magnesiumstearat, Lactose, Cellulose, Calciumphosphat, Talkum usw., enthalten sein; ihr Anteil kann z.B. zwischen 10 und 50 Gew.-% liegen. Vorzugsweise enthält die Pulvermischung zur Herstellung der verpreßten Matrix zusätzlich eine von den genannten Acryl- und Vinylesterpolymeren verschiedene, in Wasser und Magensaft schwer lösliche Polymersubstanz, wie Ethylcellulose, Celluloseacetat, Celluloseacetatphthalat, Hydroxypropylmethylcellulosephthalat oder Polyvinylacetat. Die Mischung wird in an sich bekannter Weise, üblicherweise bei Preßkräften zwischen 2 und 30 kN, zu Komprimaten, wie Tabletten oder Mikrotabletten, von 0,1 bis 30 mm Durchmesser verpreßt.

**Ausführung des Verfahrens**

Die Komprimate werden erfindungsgemäß einer Alkoholdampf enthaltenden Gasphase ausgesetzt. Damit die Gasphase schnell und gleichförmig in das Komprimat eindringen kann, ist es vorteilhaft, es zunächst einem Vakuum auszusetzen, um die Luft aus den Poren zu ziehen, und die Gasphase danach in den evakuierten Behandlungsraum einströmen zu lassen. Die Vakuumbehandlung kann gegebenenfalls ein- oder mehrmals wiederholt werden. Um einen gleichförmigen Retardierungseffekt zu erzielen, soll Sorge getragen werden, daß alle Komprimatteilchen in gleicher Weise der Gasphase ausgesetzt werden. In einer weitgehend inertgasfreien Gasphase wird diese Voraussetzung auch in einem großen Volumen einer unbewegten Schüttung von Komprimatteilchen erfüllt.

Es ist zweckmäßig, die fortschreitende Aufnahme des Alkoholdampfes anhand der Gewichtszunahme zu verfolgen und beim erwünschten Sättigungsgrad zu beenden. Sie kann bis zur vollständigen Sättigung fortgesetzt werden, die erreicht ist, wenn keine Gewichtszunahme mehr eintritt. Die Verklebung der Hilfsstoffteilchen zu einer porösen Matrix tritt gleichzeitig mit der Absorption des Alkohols ein, kann aber bei gleichbleibendem Sättigungsgrad mit der Zeit weiter fortschreiten.

Auch die Temperatur hat einen deutlichen Einfluß auf den Retardierungseffekt. Mit steigender Temperatur wird der Hilfsstoff weicher und der Dampfdruck des Alkohols höher, wodurch der Effekt beschleunigt wird. Wenn das Komprimat die gleiche Temperatur wie die Gasphase und die Behandlungsapparatur hat, wird ein gut reproduzierbares Ergebnis erzielt. Ist das Komprimat kühler als die Gasphase, kann es zu Kondensatbildung innerhalb der Komprimatteilchen und infolge davon zu einem über den Querschnitt des Komprimats ungleichförmigen Retardierungseffekt kommen. Um die Kondensatbildung in der Matrix zu unterdrücken, kann die Gasphase auf einer Temperatur oberhalb des Sättigungspunktes gehalten werden. Allgemein besteht bei Temperaturunterschieden des Komprimats, der Anlage und der Gasphase stets die Gefahr einer ungleichmäßigen und schwer reproduzierbaren Einwirkung der Gasphase. Daher sind niedrige und gleichförmige Temperaturen, besonders im Bereich von 18 bis 50°C, vorteilhaft.

Die Behandlungsdauer liegt in der Regel zwischen 5 Minuten und 24 Stunden, vorzugsweise bei 20 bis 60 Minuten. Nach ausreichender Einwirkung des absorbierten Alkohols wird die Behandlung durch Desorption abgebrochen. Dazu wird das behandelte Komprimat vorteilhaft unter gelindem Erwärmen einem Vakuum ausgesetzt, bis das ursprüngliche Gewicht nahezu wieder erreicht ist. Ein Vakuum von 2 bis 100 mbar und Behältertemperaturen von 20 bis 70°C sind im allgemeinen ausreichend. Die Desorption dauert oft länger als die Absorption, was mit der verminderten Porengröße zusammenhängt. Es gehört jedoch zu den Vorzügen der Erfindung, daß die Desorption schneller und vollständiger vonstatten geht als nach der Einwirkung von Aceton oder ähnlich guten Lösemitteln.

Sowohl bei der Absorption als auch bei der Desorption ist es vorteilhaft, die Schüttung des Komprimats gleichförmig oder intermittierend zu bewegen, beispielsweise in einer waagerecht gelagerten Drehtrommel, mittels Rührschaufeln oder in einer Wirbelschichtapparatur. Jedoch werden in den meisten Fällen bei unbewegter Lagerung einer Schüttung des Komprimats auf einem geeigneten Netzboden gleichwertige Ergebnisse erzielt.

Das Komprimat bleibt in der Regel durch die erfindungsgemäße Behandlung äußerlich unverändert. Auch an den Bruchflächen eines Komprimatteilchens läßt sich die fortschreitende "Versinterung" der Matrix meist nicht beobachten. Die Festigkeit des Komprimats nimmt durch die Behandlung in der Regel zu. Eine verläßliche Aussage über den Retardierungseffekt ist nur mit einer Freisetzungsprüfung möglich.

Aufbau und Betrieb

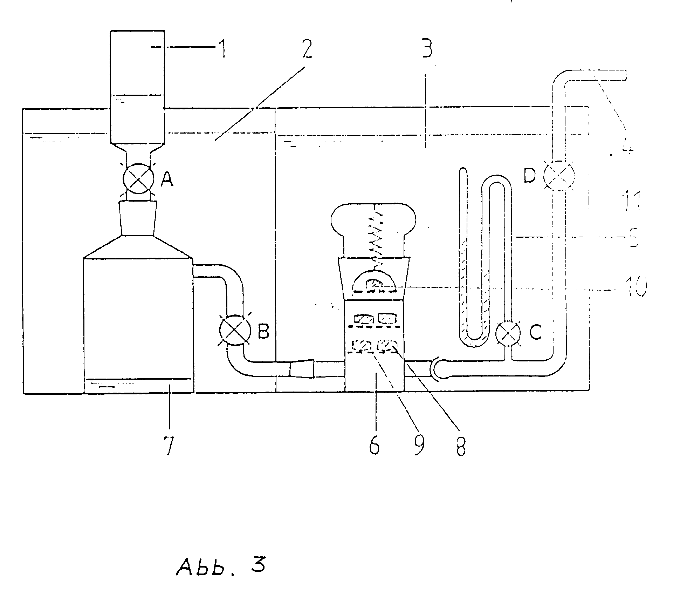

Die in dem nachfolgenden Beispielen verwendete Apparatur zur Dampfbehandlung (Abb. 3) ist in Figur 3 schematisch dargestellt und besteht aus einer Dampferzeugungseinheit und einem Inkubationsteil mit Quecksilbermanometer 5. Alle Teile sind aus Glas gearbeitet, um eine optische Kontrolle des Inkubationsprozesses durchführen zu können. Der Lösemitteldampferzeuger besteht aus einer 100 ml Glasflasche mit einer Schliffverbindung zum Tropftrichter 1 und einer zweiten Verbindung zum Inkubationsraum 6. Der Lösemitteldampf wird durch das Einbringen von 100 ml der entsprechenden Flüssigkeit erzeugt. Dazu wird die Apparatur über die Leitung 4 evakuiert und das entgaste Lösemittel so aus dem Tropftrichter eingesaugt, daß ein Luftzutritt ausgeschlossen wird. Da die Dampfgeneratoreinheit 7 im Wasserbad 2 auf 25 Grad C temperiert ist, ensteht eine gesättigte Gasphase mit dem entsprechenden Sattdampfdruck des verwendeten Lösemittels solange es im Überschuß in der Erzeugungseinheit vorhanden ist.
Die Temperatur im Inkubationsraum 6 bzw. in Wasserbad 3 wurde mit 30 Grad C fünf Grad über der Dampferzeugungstemperatur gewählt, damit durch leichte Temperaturschwankungen bedingte unkontrollierbare Kondensationserscheinungen, wie sie im Sattdampfbetrieb auftreten könnten, vermieden werden.
Im Inkubationsraum 6 werden die Tabletten 8 einschichtig auf Drahtnetzen 9 mit einer lichten Massenweite von 1 mm dem Lösemitteldampf ausgesetzt.
Zur Bestimmung der Sorptions- und Desorptionszyklen befindet sich jeweils eine Tablette 10 in einer Halterung an einer Federwaage 11.

Nachdem die beiden Temperierbäder 2 und 3 ihre Temperaturen auf 25 ± 0,5 Grad C bzw. 30 ± 0,5 Grad C konstant halten, werden folgende Arbeitsschritte nacheinander durchgeführt:

- Evakuierung des gesamten Systems bei geschlossenem Absperrhahn A (B,C,D geöffnet) über den Absaugstutzen 4 bis zu einem Restdruck unter 1 Torr. Um evtl. stattfindende Desorptionsprozesse an den Tabletten (z.B. Luft, Wasser) konstant zu halten, wird das System 30 Minuten so belassen. Gleichzeitig kann die Dichtigkeit der Anlage überprüft werden.

- Bei geschlossenem Hahn B wird durch das Vakuum aus dem Tropftrichter eine genügende Menge an entgastem und auf 25 Grad C temperiertem Lösemittel unter Vermeidung von Luftzutritt eingesaugt. In der Dampferzeugungseinheit stellt sich der Sattdampfdruck des jeweiligen Lösemittels zur Temperatur von 25 Grad C ein.

- Beginn der Inkubationszeit:

Bei geschlossenem Hahn A und D wird B geöffnet: Der Lösemitteldampf strömt in den Inkubationsraum ein; sofort stellt sich hier derselbe Dampfdruck wie in der Dampferzeugungseinheit ein.

- Ende der Inkubationszeit:

Schließen des Absperrhahnes B und Evakuierung der Inkubationseinheit:

Nach beendeter Desorption des Lösemittels (Kontrolle über Desorptionsisotherme) wird das Vakuum aufgehoben und die Tabletten können entnommen werden.

Unter den oben beschriebenen Bedingungen stellen sich bei 25 Grad C für die verschiedenen Lösemittel folgende Sattdampfdrücke ein:

| | |
|---|---|
| Ethanol: | 7.540 ± 260 Pa (58 ± 2 Torr) |
| Isopropanol: | 5.720 ± 260 Pa (44 ± 2 Torr) |
| Aceton: | 28.850 ± 260 Pa (222 ± 2 Toor) |

BEISPIELE

Beispiel 1

Eine gleichmäßige Pulvermischung aus

88 Gew.-Tln. eines Mischpolymerisats aus gleichen Gewichtsteilen Methylmethacrylat und Äthylacrylat, sprühgetrockn. Emulsionspolymerisat, Teilchengröße 2 - 20 μm,

2 Gew.-Tln. hochdisperses Siliciumdioxid (DAB 9)

10 Gew.-Tln. Kochsalz

wurde mit 9 mm-Stempeln bei einer Preßkraft von 15 kN zu Tabletten von je 100 mg verpreßt.

Die Freisetzung von Kochsalz aus den behandelten Tabletten wurde mit jeweils 2 Tabletten à 100 mg in 500 ml Wasser von 37°C in einem US Paddle-Gerät bei 50 Upm geprüft. In der nachfolgenden Tabelle sind die Zeiten angegeben und mit $t_{50\%}$ bzw. $t_{90\%}$ bezeichnete, nach denen 50 % bzw. 90 % des Kochsalzes freigesetzt waren. Ergebnisse:

| Behandlung der Tabletten | $t_{50\%}$ | $t_{90\%}$ |
|---|---|---|
| unbehandelt | 10 min | 45 min |
| 30 min Äthanoldampf | 55 min | 250 min |
| 60 min Äthanoldampf | 125 min | 520 min |
| 15 min Acetondampf | 450 min | – |

Beispiel 2

Die gleiche Pulvermischung wie im Beispiel 1 wurde mit einem 3 mm-Stempel bei 5 kN zu Tabletten von je 7 mg verpreßt und die Tabletten zum Teil wie dort mit Äthanoldampf behandelt. Die Freisetzungsgeschwindigkeit unter gleichen Prüfbedingungen betrug:

| Tablettenbehandlung | $t_{50\%}$ | $t_{90\%}$ |
|---|---|---|
| unbehandelt | 5 min | 19 min |
| 120 min Äthanoldampf | 87 min | 310 min |

**Beispiel 3**

Es wurden wie in Beispiel 1 Tabletten hergestellt aus folgenden Pulvergemisch:

| | |
|---|---|
| 50 Gew.-Tl. | Mischpolymerisat, 2 - 20 μm, aus 67 % Methylmethacrylat und 33 % Äthylacrylat |
| 5 Gew.-Tl. | Fasercellulose |
| 0,5 Gew.-Tl. | Magnesiumstearat, 25-100 μm |
| 20 Gew.-Tl. | Salicylsäure, 100 - 150 μm |
| ad 100 Gew.-Tl. | Lactosehydrat, 25 - 250 μm |

Zum Pressen wurde ein 8 mm-Stempel und eine Preßkraft von 10 kN angewendet. Das Tablettengewicht betrug 100 mg. Nach der Behandlung mit Äthanoldampf wie im Beispiel 1 wurde die Wirkstoffreisetzung im Acetat-Puffer von pH 5,0 im US Paddle-Gerät gemessen:

| Tablettenbehandlung | $t_{50\%}$ | $t_{75\%}$ |
|---|---|---|
| unbehandelt | 25 min | 90 min |
| 15 min Äthanoldampf | 230 min | 480 min |
| 30 min Äthanoldampf | 570 min | – |

**Beispiel 4**

Es wurden wie im Beispiel 3 Tabletten hergestellt aus einem Pulvergemisch aus:

| | |
|---|---|
| 50 Gew.-Tl. | Äthylcellulose (Visk.d.5% Lösung 45 mPa·s) Handelsbezeichnung "Ethocel 45 cps" |
| 10 Gew.-Tl. | Methylmethacrylat-Äthylacrylat-Mischpolymerisat |
| 5 Gew.-Tl. | Fasercellulose |
| 0,5 Gew.-Tl. | Magnesiumstearat, 25 - 100 μm |
| 20 Gew.-Tl. | Tetracain-hydrochlorid, 50 - 200 μm |
| ad 100 Gew.-Tl. | Lactosehydrat, 25 - 250 μm |

Wirkstofffreisetzung in Wasser nach Äthanoldampfbehandlung wie im Beispiel 1:

| Tablettenbehandlung | $t_{50\%}$ | $t_{90\%}$ |
|---|---|---|
| unbehandelt | 18 min | 110 min |
| 30 min Äthanoldampf | 32 min | 182 min |

**Beispiel 5**

Ein Pulvermischung aus

| | |
|---|---|
| 97 Gew.-Tl. | des Mischpolymerisats wie in Beispiel 1, |
| 3 Gew.-Tl. | Pilocarpin-hydrochlorid, 100 - 450 μm |

wurde mit speziellen nierenförmigen Stempeln mit einer Preßkraft von 10 kN zu Komprimaten von 10 mg mit 0,3 mm Steghöhe zur Anwendung im Augenlid gepreßt und die Komprimate zum Teil mit Äthanoldampf behandelt. Die Freisetzungsprüfung in Wasser ergab:

| Tablettenbehandlung | $t_{50\%}$ | $t_{90\%}$ |
|---|---|---|
| unbehandelt | 3 min | 8 min |
| 30 min Äthanoldampf | 35 min | 180 min |

Beispiel 6:

Eine Dispersion aus 80 Gew.-Teilen des Mischpolymerisates wie in Beispiel 1 und 20 Gewichtsteilen Natriumchlorid wurde sprühgetrocknet und das erhaltene Pulver mit 8 mm-Stempeln mit einer Preßkraft von 10 kN zu Komprimaten von 100 mg verpreßt. Die Freisetzungsprüfung in Wasser ergab:

| Tablettenbehandlung | $t_{50\%}$ | $t_{90\%}$ |
|---|---|---|
| unbehandelt | 40 min | 170 min |
| 30 min Isopropanoldampf | 90 min | 300 min |
| 60 min Isopropanoldampf | 110 min | 360 min |

Beispiel 7:

Eine Pulvermischung aus
79,5 Tln. eines pulverisierten Copolymerisats aus
65 Gew.-% Methylmethacrylat
30 Gew.-% Äthylacrylat
5 Gew.-% Trimethylammoniumäthylmethacrylatchlorid
0,4 Tln. Talkum
20,0 Tln. Natriumsalicylat
wird bei einem Druck zu Tabletten von 3 mm Durchmesser verpreßt. Die Tabletten werden 1 bis 6 Stunden einer Äthanoldampfatmosphäre ausgesetzt.
Die Freisetzung von Natriumsalicylat in reinem Wasser bei 37 Grad C wurde unter schwacher Rührung (50 Upm) anhand der UV-Extinktion bei 296 nm verfolgt.

| Behandlungsdauer in Äthanoldampf: | 0 | 1 | 2 | 4 | 6 | Std. |
|---|---|---|---|---|---|---|
| Freisetzung von Na-Salicylat innerhalb 2 Std. (in % des Gesamtgehalts | 100 | 97 | 90 | 80 | 60 | % |
| 90 % Freisetzung nach: | 15 | 40 | 120 | 240 | 550 | min. |

Beispiel 8:

Eine Pulvermischung bestehend aus:
50,0 Tln. eines pulverförmigen Copolymers aus
33 Gew.-% Methacrylsäure
67 Gew.-% Methylmethacrylat
5.0 Tln. Cellulosefasern
24,5 Tln. Lactose-Hydrat
0,5 Tln. Magnesiumstearat
20,0 Tln. Salicylsäure
wurde zu Tabletten mit einem Einzelgewicht von 12,5 mg und einem Durchmesser von 3 mm verpreßt. Die Tabletten wurden 1 bzw. 2 Stunden einer Äthanoldampfatmosphäre ausgesetzt. Die Freisetzung von Natriumsalicylat wurde wie im Beispiel 7 bestimmt.

```
Äthanoldampfbehandlungsdauer:          0    0.5    1    Std.

Freisetzung von 15 % der ent-
haltenen Salicylsäure:
a) in Acetatpuffer pH 5,0 nach         80   160   550   min.
b) in Phosphatpuffer pH 7,4 nach       45    85   280   min.
```

## Patentansprüche

1. Verfahren zur Herstellung von Arzneiformen mit einer Wirkstoff und einen Hilfsstoff enthaltenden Matrix zur verzögerten Wirkstofffreisetzung, wobei eine zu einer festen Matrix verpreßte Mischung aus einem pulverförmigen Wirkstoff und einem pulverförmigen Acrylesterpolymer oder Vinylesterpolymer, das in der am Anwendungsort vorliegenden Körperflüssigkeit nicht löslich ist, als Hilfsstoff einer Gasphase ausgesetzt wird, die den Dampf eines organischen Lösungsmittels enthält,
   dadurch gekennzeichnet,
   daß die gepreßte Matrix einer überwiegend aus dem Dampf eines niederen Alkohols bestehenden Gasphase ausgesetzt wird.

2. Verfahren nach Anspruch 1, dadurch gekennzeichnet, daß als Acrylpolymer ein Homo- oder Copolymerisat aus wenigstens 30 Gew.-% eines Alkylesters der Acryl- oder Methacrylsäure mit 1 bis 8 C-Atomen im Alkylrest und bis zu 70 Gew.-% eines damit mischpolymerisierbaren, alpha-beta-äthylenisch ungesättigten Comonomeren eingesetzt wird.

3. Verfahren nach Anspruch 2, dadurch gekennzeichnet, daß als Acrylpolymer ein Copolymerisat eingesetzt wird, das als Comonomerkomponente Einheiten von Acryl- oder Methacrylsäure und/oder von deren Hydroxyalkylestern enthält, wobei deren Alkylreste 2 bis 6 C-Atome enthalten.

4. Verfahren nach einem der Ansprüche 1 bis 3, dadurch gekennzeichnet, das als niederer Alkohol Äthanol in der Dampfform eingesetzt wird.

5. Verfahren nach einem der Ansprüche 1 bis 4, dadurch gekennzeichnet, daß eine verpreßte Matrix, die zusätzlich eine von den genannten Acryl- und Vinylesterpolymeren verschiedene, in Wasser und Magensaft schwer lösliche Polymersubstanz enthält, eingesetzt wird.

6. Verfahren nach Anspruch 5, dadurch gekennzeichnet, daß als zusätzliche Polymersubstanz Ethylcellulose, Celluloseacetat, Celluloseacetatphthalat, Hydroxypropylmethylcellulosephthalat oder Polyvinylacetat verwendet wird.

7. Arzneiform mit retardierter Wirkstofffreisetzung, enthaltend eine aus einem im Magensaft nicht löslichen pulverförmigen Acrylesterpolymer oder Vinyesterpolymer als Hilfsstoff und einem pulverförmigen Wirkstoff gepreßte Matrix, die einer Gasphase ausgesetzt worden ist, die den Dampf eines organischen Lösungsmittels enthält, dadurch gekennzeichnet, daß die gepreßte Matrix zum Erreichen einer verzögerten Wirkstofffreisetzung einer überwiegend aus dem Dampf eines niederen Alkohols bestehenden Gasphase ausgesetzt worden ist.

8. Arzneiform nach Anspruch 7 in Form einer Tablette.

## Claims

1. A process for preparing a pharmaceutical preparation having a matrix containing an active substance and an excipient for the delayed release of the active substance, whereby a mixture, compressed to form a

9

firm matrix consisting of a powdered active substance and a powdered acrylic ester polymer or vinyl ester polymer as excipient, which is insoluble in the body fluid present at the site of application, is subjected to a gaseous phase containing the vapour of an organic solvent, characterised in that the compressed matrix is subjected to a gaseous phase which largely consists of the vapour of a lower alcohol.

2. A process according to claim 1, characterised in that a homo- or copolymer consisting of at least 30 wt.% of an alkyl ester of acrylic or methacrylic acid having 1 to 8 carbon atoms in the alkyl group and up to 70 wt.% of an alpha-beta-ethylene-unsaturated comonomer copolymerisable therewith are used as acrylic polymer.

3. A process according to claim 2, characterised in that a copolymer is used as acrylic polymer, said copolymer containing, as comonomer component, units of acrylic or methacrylic acid and/or of the hydroxyalkylesters thereof, in which the alkyl groups contain 2 to 6 carbon atoms.

4. A process according to claims 1 to 3, characterised in that ethanol in the vapour form is used as the lower alcohol.

5. A process according to claims 1 to 4, characterised in that a compressed matrix is used which additionally contains a polymer substance different from the said acrylic and vinyl ester polymers and not easily soluble in water and gastric fluid.

6. A process according to claim 5, characterised in that ethylcellulose, cellulose acetate, cellulose acetate phthalate, hydroxypropyl methyl cellulose phthalate, or polyvinyl acetate are used as additional polymer substance.

7. A pharmaceutical preparation with delayed release of active substance, containing a matrix compressed from a powdered acrylic ester polymer or vinyl ester polymer as excipient, which is not soluble in the gastric juice, and a powdered active substance, said matrix having been subjected to a gaseous phase containing the vapour of an organic solvent, characterised in that the compressed matrix has been subjected to a gaseous phase largely consisting of the vapour of a lower alcohol in order to achieve a delayed release of active substance.

8. A pharmaceutical preparation according to claim 7 in the form of a tablet.


## Revendications

1.- Procédé de préparation de formes médicamenteuses présentant une matrice qui contient la substance active et un adjuvant pour la libération retardée de la substance active, un mélange d'une substance active en poudre et d'un polymère d'ester acrylique ou d'ester vinylique en poudre, qui n'est pas soluble dans le liquide corporel présent au lieu d'administration, étant pressé en une matrice solide et étant exposé à une phase gazeuse qui sert d'adjuvant et qui contient la vapeur d'un solvant organique, caractérisé en ce que la matrice pressée est exposée à une phase gazeuse qui se compose principalement de la vapeur d'un alcool inférieur.

2. - Procédé selon la revendication 1, caractérisé en ce qu'on utilise, comme polymère acrylique, un homo- ou copolymère d'au moins 30% en poids d'un ester alkylique de l'acide acrylique ou méthacrylique contenant 1 à 8 atomes de carbone dans le reste alkyle, et de 70% en poids au maximum d'un comonomère à insaturation $\alpha,\beta$-éthylénique, copolymérisable avec cet ester.

3. - Procédé selon la revendication 2, caractérisé en ce qu'on utilise, comme polymère acrylique, un copolymère qui contient, en tant que composant comonomère, des motifs d'acide acrylique ou méthacrylique et/ou de leurs esters hydroxyalkyliques les restes alkyle de ces esters contenant de 2 à 6 atomes de carbone.

4.- Procédé selon l'une quelconque des revendications 1 à 3, caractérisé en ce qu'on utilise, comme alcool inférieur, de l'éthanol sous forme de vapeur.

5.- Procédé selon l'une quelconque des revendications 1 à 4, caractérisé en ce qu'on utilise une matrice pressée qui contient en plus une substance polymère différente desdits polymères d'ester acrylique et vinylique, difficilement soluble dans l'eau et le suc gastrique.

6. - Procédé selon la revendication 5, caractérisé en ce qu'on utilise, comme substance polymère supplémentaire, de l'éthylcellulose, de l'acétate de cellulose, de l'acétate-phtalate de cellulose, du phtalate d'hydroxypropylméthylcellulose ou de l'acétate de polyvinyle.

7.- Forme médicamenteuse à libération retardée de la substance active, contenant une matrice pressée à partir d'un polymère d'ester acrylique au d'ester vinylique en poudre, non soluble dans le suc gastrique, en tant qu'adjuvant, et d'une substance active en poudre, matrice qui a été exposée à une phase gazeuse qui contient la vapeur d'un solvant organique, caractérisée en ce que, pour parvenir à une libération retardée de la substance active, la matrice pressée a été exposée à une phase gazeuse qui se compose principalement de la vapeur d'un alcool inférieur.

8.- Forme médicamenteuse selon la revendication 7 sous forme d'un comprimé.

Sorption und Desorption verschiedener Loesungsmittel
an PMMA DN sd Tbl., Ø: 8mm, 100mg, 15 kN

Sorption und Desorption verschiedener Lösungsmittel
an Tabletten mit 8mm Durchmesser und 100 mg Gewicht
gepreßt mit 15 kN; Zusammensetzung wie Beispiel 1
Abb. 1

Zeit (min)

Freisetzung von NaCl aus PMMA ON sd Tabletten

USP Paddle, 50 Upm, Wasser 500ml, 37°C

100mg, 10% NaCl, 9mm, 1.5mm Steghoehe

Abb.2

Abb. 3